# EUROPÄISCHE PATENTSCHRIFT

(11) **EP 2 051 955 B1**
(45) Veröffentlichungstag und Bekanntmachung des Hinweises auf die Patenterteilung: **31.08.2016**
(21) Anmeldenummer: 07787858.5
(22) Anmeldetag: 24.07.2007
(51) Int. Cl.: C07C 45/75, C07C 49/82, C07C 49/245, C07C 49/337, C07C 49/17, C07C 319/20, C07C 323/22, C07D 233/28, C07D 233/90, C07D 307/46, B01J 31/02

(54) **HERSTELLUNG VON ALPHA-HYDROXYKETONEN ÜBER CARBEN-KATALYSIERTE UMPOLUNGSREAKTION VON ALDEHYDEN**
PRODUCTION OF ALPHA HYDROXY KETONES VIA CARBENE CATALYZED POLE REVERSAL REACTION OF ALDEHYDES
FABRICATION D'ALPHA-HYDROCETONES PAR REACTION D'INVERSION DE POLARITE D'ALDEHYDES, CATALYSEE PAR CARBENE

(30) Priorität: 18.08.2006 DE 102006038934
(43) Veröffentlichungstag der Anmeldung: 29.04.2009
(73) Patentinhaber: Evonik Degussa GmbH, 45128 Essen (DE)
(72) Erfinder: JACKSTELL, Ralf, 18106 Rostock (DE); JOVEL, Irina, 18106 Rostock (DE); BELLER, Matthias, 18211 Ob Nienhagen (DE); HATELEY, Martin, 81825 München (DE); WECKBECKER, Christoph, 63584 Gründau-lieblos (DE); HUTHMACHER, Klaus, 63571 Gelnhausen (DE)
(86) Internationale Anmeldenummer: PCT/EP2007/057626
(87) Internationale Veröffentlichungsnummer: WO 2008/019927

(56) Entgegenhaltungen:
- EP-A- 0 580 956
- WO-A1-2008/010609
- W SCHÖSSLER , M REGITZ: "Stabile Dipole aus 1,1',3,3'-Tetraphenyl-2,2'-biimidazolidiny liden und Acylciso- bzw. Acylisothiocyanaten" CHEM BER, Bd. 107, 1974, Seiten 1931-1948, XP009091637 in der Anmeldung erwähnt
- H A DUONG ET AL.: "Reversible carboxylation of N-heterocyclic carbenes" CHEM COMMUN, 2004, Seiten 112-113, XP002457186 in der Anmeldung erwähnt
- I TOMMASI, F SORRENTINO: "Synthesis of 1,3-dialkylimidazolium-2-carboxylates by direct carboxylation of 1,3-dialkylimidazolium chlorides with CO2" TETRAHEDRON LETTERS, Bd. 47, 4. August 2006 (2006-08-04), Seiten 6453-6456, XP002457187
- TUDOSE A ET AL: "Imidazol(in)ium-2-carboxylates as N-heterocyclic carbene precursors in ruthenium-arene catalysts for olefin metathesis and cyclopropanation", JOURNAL OF ORGANOMETALLIC CHEMISTRY, ELSEVIER-SEQUOIA S.A. LAUSANNE, CH, vol. 691, no. 24-25, 1 December 2006 (2006-12-01), pages 5356-5365, XP028048267, ISSN: 0022-328X, DOI: 10.1016/J.JORGANCHEM.2006.07.035 [retrieved on 2006-12-01]

## Beschreibung

Die vorliegende Erfindung bezieht sich auf die Herstellung von α-Hydroxyketonen der allgemeinen Formel I und im Falle wenn R'=R=H deren anschließenden Oxidation zu α-Ketosäuren.

Insbesondere bezieht sich die vorliegende Erfindung auf neue Imidazoliniumcarboxylat-Addukte sowie ein neues Verfahren zur Anwendung katalytischer Mengen Imidazolium-und Imidazoliniumcarboxylat-Addukte in der Acyloinreaktion (Umpolungsreaktion von Aldehyden) zur Herstellung von Hydroxyketonen der allgemeinen Formel I, 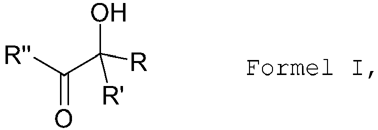 wobei R und R' gleich oder verschieden sind und H oder einen geradkettigen oder verzweigten und ggf. substituierten C₁-C₁₂-Alkyl-Rest bedeuten und R" = H₃CSCH₂CH₂, t-Butyl, n-Butyl, sek-Butyl, n-Propyl, i-Propyl, ggf. Heteroatom-substituiertes C₆-C₁₈-Aryl, Heteroaryl, C₆-C₁₈-Arylalkyl, insbesondere Phenylmethyl, wobei Phenyl wieder heteroatomsubstitutiert sein kann, oder Heteroalkyl ist.

**α-Hydroxyketone** sind wegen ihrer Funktionalität wichtige Synthesebausteine für eine Vielzahl von Chemikalien, zum Beispiel:
- zur Herstellung von Heterocyclen wie Imidazolen (EP-A 252162) und Imidazolonen (Journal of the Chemical Society Perkin II 1981, 310), die Vorstufen zu Arzneiund Pflanzenschutzmitteln darstellen,
- als Reduktionsmittel in der Färberei zum Färben von Textilen wegen ihrer Reduktionsvermögens (EP-A 364752),
- als Aromastoff in Lebensmitteln, wie Acetoin oder das daraus resultierende Diacetyl,
- und sie sind darüber hinaus wichtig als Strukturmotiv und häufiger Bestandteil in Naturprodukten, was für zukünftige Arzneimittel von großer Bedeutung sein dürfte (Journal of the American Chemical Society, 2004, 3070).

**α-Ketosäuren** können aus α-Hydroxyketonen der allgemeinen Formel I mit R' = R = H durch eine geeignete Oxidation der Alkohol-Funktionalität hergestellt werden. Unter anderem werden α-Ketosäuren als Pharmaprodukte und Vorstufen verwendet.

Darüber hinaus könnte man die Ketosäure-Vorstufen der wichtigen Produkte Methionin oder Methioninhydroxyanaloges (MHA) durch eine geeignete Oxidation der α-Hydroxyketone der allgemeinen Formel I wo R'= R= H und R'' = CH₃S (CH₂)₂ herstellen. Der entscheidende Vorteil dabei wäre, dass die hochgiftige und gefährliche bisher verwendete Blausäure (HCN) durch den weit weniger gefährlichen Formaldehyd (HCHO) ersetzt werden könnte.

Es ist dem Fachmann bekannt, dass α-Hydroxyketone der allgemeinen Formel I 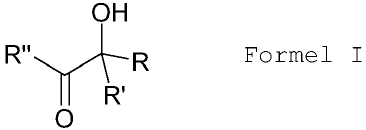 auf verschiedene Art und Weise hergestellt werden können, z. B. durch

**Benzoin-Reaktion:** Darunter versteht man die Addition von zwei Aldehyden zu einem α-Hydroxyketon durch eine Umpolung eines Aldehyds mit Cyanid als Katalysator. Wegen der Stabilisierung durch den aromatischen Ring ist die Reaktion mit Cyanid auf aromatische Aldehyde beschränkt(Organische Chemie, K. Peter, C. Vollhardt VCH, s. 1025, und auch Castells et al. Tetrahedron letters 1985, 26, 5457). Auch seit längerem bekannt ist die Thiazoliumcarben-katalysierte Benzoin-Kondensation (Breslow, Journal of the American Chemical Society 1959, 3719).

**Stetter Reaktion:** Darunter versteht man die Addition von einem umgepoltem Aldehyd mit einem 1,4-Elektrophil, die mit Carben katalysiert werden kann.

**Als Carben-Katalysatoren** sind verschiedene, N-heterocyclische Carbene bekannt und vielseitig einsetzbar - z.B. als Liganden für Übergangsmetalle, als nucleophile Katalysatoren für Acylierungen, Umesterungen oder ringöffnende Polymerisationen. Als Carben-Katalysator-Klassen für die Umpolung von Aldehyden finden die aus der Natur bekannten Thiazoliumcarbene, die Imidazoliumcarbene und die Triazoliumcarbene Verwendung.

Die aktiven Carben-Katalysatoren sind normalerweise gegen Wasser und Luft empfindlich. Sie werden aus dem entsprechenden Imidazolium-, Thiazolium- oder Triazolium-Salz durch Deprotonierung mit einer Base erzeugt. Als Basen werden u.a. Natriumhydrid, Kaliumhydrid oder Kalium-tert.-butylat in THF verwendet (Nair et al., Angewandte Chemie, 2004, Vol. 116, 5240 ff.).

Es ist aber auch bekannt, dass man katalytische Mengen der aktiven Katalysatoren in situ durch die Anwendung eines Zweiphasensystems erzeugen kann Waymouth et al., Journal of the American Chemical Society, 2003, 3046).

**Carben-Carboxylate/CO₂-Addukte:** Die Tabelle 1 aufgeführten CO₂-Addukte der Imidazolium- bzw. Imidazoliniumcarbene 1-7 sind bekannt.

**Tabelle 1: Bekannte CO₂-Addukte von Imidazolinium- und Imidazoliumsalzen**

| Struktur (Verbindungsnr.) | Name | Lit. |
|---|---|---|
| 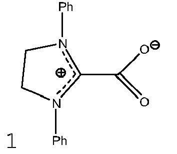 | 1, 3-Diphenylimidazolini um-2-carboxylat | W. Schössler, M. Regitz. Chem. Ber. 1974,107,1931-1948. |
| 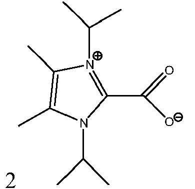 | 1,3-Diisopropyl-4,5-dimethyl-1H-imidazol-3-ium-2-carboxylat | N. Kuhn, T. Kratz. Synthesis 1993, 561-562. N. Kuhn, M. Steimann, G. Weyers. Z. Naturforsch. 1999, 54b, 427-433. |
| 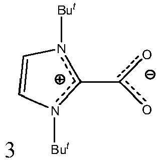 | 1,3-Di-tert-butylimidazolium-2-carboxylat | K. Ishiguro, K. Hirabayashi, T. Nojima, Y. Sawaki. Chem. Lett. 2002, 796-797. |
| 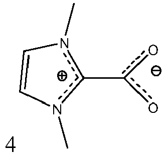 | 1, 3-Dimethylimidazolium -2-carboxylat | J. D. Holbrey, W. M. Reichert, I. Tkatchenko, E. Bouajila, O. Walter, I. Tommasi, R. D. Rogers. Chem. Comm., 2003, 28-29. |
| 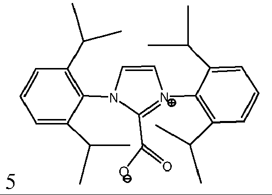 | 1,3-Dimesityl-1H-imidazol-3-ium-2-carboxylat | H. A. Duong, T. N. Tekavec, A. M. Arif, J. Louie. Chem. Comm., 2004, 112-113. |
| 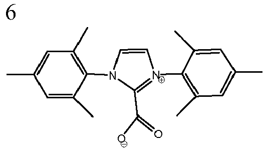 | 1,3-bis(2,6-Diisopropylphenyl)-1H-imidazol-3-ium-2-carboxylat | |
| IMes.CO₂ | | |
| 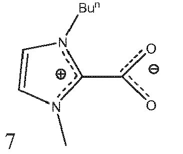 | 3-Tert-butyl-1-methyl-1H-imidazol-3-ium-2-carboxylat | I. Tommasi, F. Sorrentino. Tetrahedron Letters, 46 (2005) 2141-2145. |

Die Verbindungen 5 und 6 wurden für die Herstellung von oligomeren und polymeren Isocyanaten, insbesondere Uretdionen und Isocyanurate verwendet (WO 2005-113626). Weitere Carboxylataddukte von der Imidazolium -und Imidazoliniumstruktur bzw. mögliche katalytische Anwendungen sind bisher nicht bekannt.

Tudose et al. Journal of organometallic chemistry, Bd. 691, Nr. 24-25, offenbart die Herstellung von Imidazolinium-2-carboxylat-Verbindungen gemäß der u.g. Formel IV, wobei R⁷ und R⁸ Wasserstoff und R⁵ und R⁶ substituiertes Phenyl bedeuten (vgl. die Herstellungsverfahren 2.2., 2.2.4. und 2.2.5.). Diese erfolgt mittels Umsetzung mit CO₂ in Anwesenheit einer starken Base wie Kalium bis(trimethylsilyl)amid oder Kalium tert-butylat. Die Verbindungen dienen als Katalysatoren für die Olefin-Metathese und Cyclopropanierung.

Die **Nachteile im Stand der Technik** zur Acyloinbildung aus Aldehyden stellen sich wie folgt dar:
- Die Verwendung von Basen bei Acyloinbildungsreaktionen, z.B. zur Freisetzung eines katalytisch aktiven Imidazoliumcarbens, verursacht Nebenreaktionen wie die Aldol-Kondensation zwischen Aldehyden und
- führt zu einer Verringerung der Ausbeute an gewünschtem α -Hydroxyketon (Acyloin).
- Die verwendeten Basen müssen anschließend vom Produkt abgetrennt werden, was zusätzlichen Aufwand bedeutet und
- zu Schwierigkeiten bei der Erzielung von hohen Reinheiten - wie sie z.B. für Pharmaprodukte erforderlich sind - führen kann.
- Darüber hinaus ist bis heute kein universelles Syntheseschema für gekreuzte Aldehyd-Aldehyd Additionen bekannt, bei denen verschiedene Aldehyde miteinander umgesetzt werden (vgl. Angewandte Chemie, 2004, 1348).
- Um Imidazoliumcarbencarboxylate II bzw. Imidazolinium carboxylate III als Quelle für die eigentlich aktiven Imidazolium- bzw. Imidazoliniumcarben-Katalysatoren einzusetzen ist zunächst eine Decarboxylierung notwendig.
- Die Decarboxylierung von solchen z.B. ungesättigten Imidazoliumcarbencarboxylaten erfolgt gemäß H.A.Duong et al. (vgl. oben) allerdings erst bei Temperaturen ab 187°C, so dass die Freisetzung des katalytisch aktiven Carbens erst bei solchen Temperaturen zu erwarten war. Dieses Ergebnis hätte den Fachmann abgehalten, die Imidazoliumcarbencarboxylate als Katalysatoren bei der Acyloinbildung ernsthaft in Erwägung zu ziehen, aufgrund der ausgeprägten Nebenreaktionen bei derartig hohen Temperaturen in der Acyloinbildungsreaktion.

Es war **Aufgabe** dieser Erfindung, geeignete Katalysatoren für ein Verfahren zur Herstellung von α-Hydroxyketonen mittels Acyloinreaktion bereitzustellen, bei dem die in-situ-Verwendung von Basen nicht notwendig ist sowie ein entsprechendes Verfahren. Insbesondere war es die Aufgabe, stabile, gut handhabbare Carben-Katalysatoren bzw. die aktiven Carben-Katalysatoren generierenden Verbindungen aufzufinden, welche eine Acyloinreaktion in einfacher Weise und unter milden Bedingungen ermöglichen und insbesondere die Nachteile des Standes der Technik vermeiden. Eine weitere Aufgabe der Erfindung war es, ein verbessertes Verfahren insbesondere für die Synthese von α-Ketoalkoholen, α-Ketoaldehyden oder α-Ketosäuren als Vorstufen in der Synthese für Pharmazeutika oder für Methionin bzw. für Methioninhydroxyanaloges (MHA) zu finden, das die genannten Nachteile des Standes der Technik vermeidet.

### Beschreibung der Erfindung

Dadurch, dass man Verbindungen der allgemeinen Formel III: 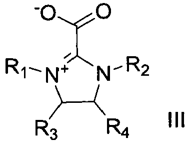 als Katalysatoren zur Herstellung von α-Hydroxyketonen mittels Acyloinaddition von Aldehyden verwendet, wobei R¹ und R² gleich oder verschieden sind und C₆- C₁₀-Aryl, Heteroaryl, verzweigtes oder unverzweigtes C₁- C₁₀- Alkyl, welches ggf. ein- oder mehrfach mit C₁-C₄-Alkyl oder mit Heteroalkyl substituiert ist, C₃-C₆-Cycloalkyl oder ein-oder mehrfach mit C₁-C₄-Alkyl substituiertes C₆-C₁₀-Aryl bedeuten und R³ und R⁴ gleich oder verschieden sind und Wasserstoff, ggf. verzweigtes C₁-C₆-Alkyl, C₃-C₆-Cycloalkyl-, C₆- C₁₀- Aryl, oder Heteroaryl bedeuten, gelingt es, die Nachteile des Standes der Technik zu überwinden.

Unter den Heteroarylresten werden Pyridyl-, Chinolyl-, Isochinolyl-, Imidazolyl-, Pyrrolyl und Furylreste bevorzugt.

Insbesondere gelingt es, die Anwesenheit von Basen während der Acyloinreaktion völlig zu vermeiden.

Wie die Beispiele und Vergleichsbeispiele in Tabelle 4 zeigen führt die Verwendung der Imidazoliniumcarben-carboxylat-Addukte als Katalysatoren durchweg zu höheren Ausbeuten an Acyloinprodukt im Vergleich zu den Imidazoliniumsalzen aus denen die aktiven Carbenkatalysatoren erst durch Baseneinwirkung freigesetzt werden.

Die erfindungsgemäße Verwendung der Imidazolium- und Imidazoliniumcarben-Carboxylate als Katalysatoren bewirkt offenbar, dass durch Abspaltung von CO₂ während der Acyloinreaktion immer eine ausreichende Menge an der eigentlich katalytisch wirksamen Carbenspezies vorhanden ist. Dies gelingt in so hervorragender Weise nur durch Einsatz von Katalysatoren der Formel III. Dabei scheint das Vorhandensein einer gesättigten C-C-Bindung zwischen den beiden benachbarten C-Atomen im Ring (Imidazolinium-Struktur III) bei Acyloinkondensationen mit Methylmercaptopropionaldehyd(MMP) von besonderer Bedeutung zu sein, da mit einer entsprechenden ungesättigten Imidazolium-Verbindung vom Typ II - wie die Erfinder herausgefunden haben keine vergleichbar guten Ergebnissse erzielt werden konnten wie insbesondere der Vergleich der Beispiele Nr. 31 und 32 zeigt (siehe Tabelle 3).

Im Gegensatz zu der gemäß H.A.Duong erst ab 187°C stattfindenden Decarboxylierung der ungesättigten Imidazoliumcarben-Carboxylate (vgl. oben), wurde hier überraschenderweise gefunden, dass Imidazolium- und Imidazoliniumcarben-Carboxylate der Formel II und III in Anwesenheit von Aldehyden bereits bei deutlich niedrigeren Temperaturen von 0 °C bis ca. 100°C ausreichend decarboxylieren und einen aktiven Carben-Katalysator generieren.

Die erfindungsgemäßen Carbencarboxylate können daher bereits bei Temperaturen von -20 °C bis 100°C für die Umsetzung von Aldehyden zu Acyloinen eingesetzt werden. Vorzugsweise wird diese Reaktion jedoch bei Temperaturen von 15 °C bis ca. 80 °C, besonders bevorzugt bei Temperaturen von 10 bis 60°C, am einfachsten jedoch bei Raumtemperatur durchgeführt.

Die Reaktion wird bevorzugt unter einem Partialdruck von 0, 1-20 bar Kohlendioxid gefahren.

Gegenstand der vorliegenden Erfindung sind daher auch Verbindungen der Formel IV, welche eine Auswahl aus den Verbindungen der Formel III darstellen: 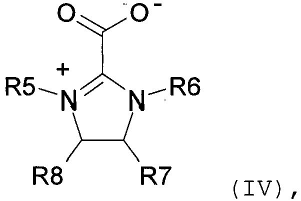 wobei R⁵ und R⁶ gleich oder verschieden sind und C₆-C₁₀-Aryl, ggf. ein oder mehrfach mit C₁-C₄-Alkyl substituiertes C₁-C₁₀- Alkyl, oder C₃-C₆-Cycloalkyl bedeuten und R⁷ und R⁸ gleich oder verschieden sind und Wasserstoff oder ggf.verzweigtes C₁-C₆-Alkyl, bevorzugt Methyl, bedeuten, mit der Maßgabe, dass wenn R⁷ und R⁸ gleich Wasserstoff sind, R⁵ und R⁶ nicht gleichzeitig Phenyl sein dürfen. Eine besonders bevorzugte Verbindung ist die Verbindung H₂IMes.CO₂ mit R⁵ = R⁶ = Mesityl und R⁷ = R⁸ = Wasserstoff. Gegenstand der vorliegenden Erfindung ist desweiteren die Verwendung von Verbindungen der allgemeinen Formel IV als Katalysatoren zur Herstellung von α-Hydroxyketonen mittels der o.g. Acyloinaddition.
Gegenstand der vorliegenden Erfindung ist auch ein Verfahren zur Herstellung von α-Hydroxyketonen der allgemeinen Formel I: 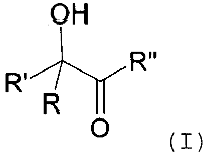 durch Umsetzung einer Carbonylverbindung der allgemeinen Formel RR' C=O mit einer Carbonylverbindung der allgemeinen Formel HR" C=O, in Gegenwart eines erfindungsgemäßen Katalysators der allgemeinen Formeln III oder IV (wie vorstehend definiert), wobei R und R' gleich oder verschieden sind und H oder einen geradkettigen oder verzweigten und ggf. substituierten C₁-C₁₂-Alkyl-Rest bedeuten und R" = H₃CSCH₂CH₂, t-Butyl, n-Butyl, sek-Butyl, n-Propyl, i-Propyl, ggf. Heteroatom-substituiertes C₆-C₁₈-Aryl, Heteroaryl, C₆-C₁₈-Arylalkyl, insbesondere Phenylmethyl, wobei Phenyl wieder heteroatomsubstitutiert sein kann, oder Heteroalkyl ist.

Unter Heteroalkyl sind in diesem Zusammenhang insbesondere primäre, sekundäre oder tertiäre Aminreste mit insgesamt 1 bis 12 C-Atomen sowie Ether- oder Thioethergruppen mit je insgesamt 1 bis 12 C-Atomen zu verstehen, die über ein C-Atom gebunden sind.

Bevorzugt sind dabei Verfahren bei denen Aldehyde mit
R = R' = H und
R'' = CH₃SCH₂CH₂ also CH₂=O und Methylmercaptopropionaldehyd eingesetzt werden. Diese Verfahren eignen sich in hervorragender Weise zur Herstellung von Vorstufen wie Verbindung V für Methionin bzw. Ketomethionin oder auch für das Methioninhydroxyanaloge (MHA), die allesamt in der Tierernährung Verwendung finden.

Die dabei notwendige Schlüsselreaktion ist die selektive Oxidation der entsprechenden Acyloinverbindung der Formel I zur Ketosäure (V), welche direkt verwendet werden kann, oder durch einfache Reduktion oder reduktive Aminierung in das MHA bzw. in Methionin umgewandelt werden kann:

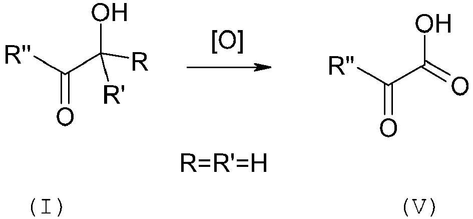

Ebenso bevorzugt ist ein Verfahren bei dem Aldehyde mit
R = H,
R' = CH₃SCH₂CH₂ und
R'' = H eingesetzt werden, so dass ein Verbindung mit endständiger Aldehydgruppe und C(-Hydroxylfunktion (VII) entsteht.

Ob die Verbindung VI oder VII entsteht, kann grundsätzlich durch eine geeignete Wahl der Reaktionsbedingungen beeinflusst werden. Die Bildung von VI scheint in der Regel jedoch bevorzugt zu erfolgen.

Auch bevorzugt ist ein Verfahren bei dem Aldehyde mit
R = H
R' = CH₃ oder C₂H₅ und
R'' = CH₃SCH₂CH₂ verwendung finden.

Bei den vorgenannten erfindungsgemäßen Verfahren werden die als Katalysatoren eingesetzten Imidazolium-Imidazoliniumcarben-Verbindungen (II) und (III) vorzugsweise in einer Konzentration von 0,1-5 Mol% bezogen auf den jenigen Aldehyd verwendet, welcher ggf. im Unterschuß eingesetzt wird.

Die erfindungsgemäß geeigneten Lösungsmittel für die Acyloinbildungsreaktion sind C₅- bis C₈-Kohlenwasserstoffe, besonders bevorzugt Heptan, aromatische Kohlenwasserstoffe, bevorzugt Toluol, Benzol bzw. Xylol, oder lineare und cyclische Ether, bevorzugt THF, Diethylether und Dioxan.

Ebenso Gegenstand der vorliegenden Erfindung ist ein Verfahren zur Herstellung von Katalysatoren der allgemeinen Formeln III oder IV 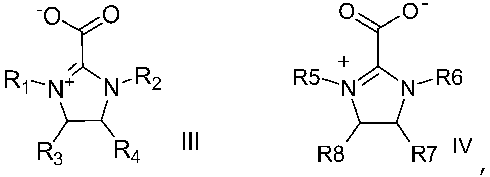 das dadurch gekennzeichnet ist, dass man eine Verbindung der allgemeinen Formel X oder XI 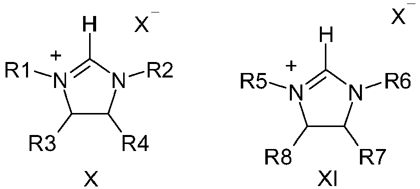 in Gegenwart einer Base mit CO₂ umsetzt, wobei X = Cl, Br, I, pCH₃C₆H₄SO₃, BF₄, PF₆, CH₃SO₃ ist.

Als Base werden vorzugsweise Verbindungen verwendet deren korrespondierende Säure einen pKa > 8 aufweist. Dabei werden vorzugsweise Alkali- und Erdalkalicarboxylate, besonders bevorzugt Natriumacetat, Alkali- und Erdalkalialkoholate, besonders bevorzugt Kaliumtertbutylat, Alkaliund Erdalkalicarbonate oder primäre-, sekundäre-, oder tertiäre-Amine sowie bicyclische Amine, vorzugsweise 1,8-Diazobicyclo[5.4.0]undec-7-ene (DBU), eingesetzt.

Die Umsetzung wird in der Regel bei Temperaturen von -20°C bis 100°C durchgeführt. Vorzugsweise wird diese Reaktion jedoch bei Temperaturen von 15°C bis ca. 80°C, besonders bevorzugt bei Temperaturen von 10 bis 60°C, am einfachsten jedoch bei Raumtemperatur durchgeführt.

Bevorzugte Lösungsmittel sind dabei THF, Diethylether oder Toluol.

Die Isolierung und Reinigung der erfindungsgemäßen Imidazoliniumcarben-Carboxylate kann dabei vorteilhaft durch Kristallisation aus Alkoholen geschehen.

Wichtige **Vorteile** der vorliegenden Erfindung sind:
- Es entstehen weniger Nebenprodukte durch Verzicht auf den Einsatz von Base während der Acyloinbildung. Die unerwünschte Aldol-Kondensation wird praktisch vollkommen vermieden.
- Es müssen keine zusätzlichen Hilfsstoffe wie die besagten Basen verwendet werden und deren Abtrennung nach der Reaktion erübrigt sich.
- Es wird eine höhere Reinheit der Acyloinprodukte erreicht im Vergleich zu herkömmlichen Verfahren.
- Das erfindungsgemäße Verfahren zeichnet sich durch sehr günstige Verfahrensbedingungen, wie niedrige Temperaturen und geringen Katalysatoreinsatz, aus.
- Durch das erfindungsgemäße Verfahren werden einige neue für die Futtermitteladditiv-Industrie wichtige Verbindungen zugänglich gemacht. Diese können wirtschaftlich und in guten Ausbeuten hergestellt werden.

### Beispiele und Vergleichsbeispiele*)

### Allgemeine Versuchsbeschreibung bezogen auf die Beispiele 1 - 44:

Es wurden Paraformaldehyd und Katalysator in einem inerten Schlenkkolben eingewogen und das Lösungsmittel (absolutes Tetrahydrofuran, THF) hinzugegeben. Anschließend wurden das Edukt (Aldehyd), der Standard für GC Analyse (Toluol, 0.1 equiv. zum Edukt) und gegebenenfalls eine Base (im Falle der Verwendung der Imidazolinium-Salze als Carbenkatalysatorvorstufe = Vergleichsbeispiele) durch ein Septum zugegeben. Das Reaktionsgemisch wurde mittels Magnetrühren ca. 30 min. gerührt. Die Reaktion wurde bei Raumtemperatur (20 - 22 °C) in der in Tabelle 2-4 jeweils angegebenen Reaktionszeit durchgeführt. Nach Beendigung der Reaktion (Verfolgung des Umsatzes durch GC-FID) wurde das Produkt nach destillativer Entfernung des Solvents durch Säulenchromatographie gereinigt. Alle unten aufgeführte Produktstrukturen wurden durch GC-MS und NMR-Daten bestätigt. Die Bestimmung der Umsätze (bzw. Ausbeute) erfolgte durch eine separate Kalibrierung mit Edukten und vorher isolierten Produkten. Die Differenzen zwischen Umsatz und Ausbeuten sind größtenteils auf di-, tri- und polymerisierte Verbindungen als Nebenprodukte zurückzuführen.

**Tabelle 2**

| | | | | |
|---|---|---|---|---|
| Durchgeführte Versuche mit 3-Methylthiopropionaldehyd (MMP) in Gegenwart von verschiedenen Katalysatoren | | | | |
| 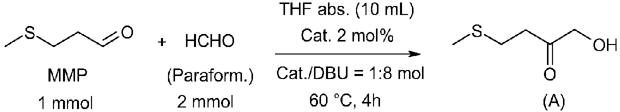 | | | | |

| **Beispiel** | **Katalysator** | **Umsatz von MMP, %** | **Hauptprodukt** | **Ausbeute (A) (GC, %)** |
|---|---|---|---|---|
| 1*) | 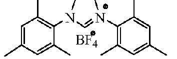 | 93 | Acyloin Kondensation | 50 |
| 2*) | 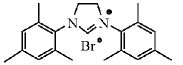 | 93 | 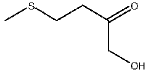 | 57 |
| 3*) | 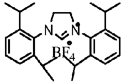 | 86 | | 44 |
| 4*) | 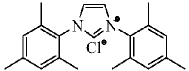 | 85 | Aldol Kondensation | 0 |
| 5*) | 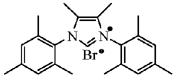 | 92 | | 0 |
| 6*) | 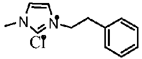 | 30 | 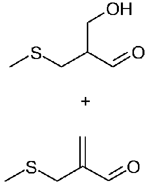 | 0 |
| 7*) | 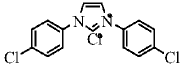 | 66 | | 0 |
| 8*) | 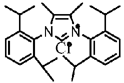 | 78 | | 0 |
| 9*) | 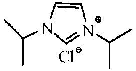 | 97 | | 0 |
| 10*) | 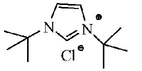 | 100 | | 0 |
| 11*) | 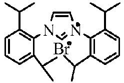 | 74 | | 0 |
| 12*) | 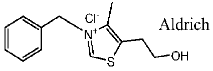 | 67 | | 0 |
| 13*) | 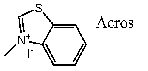 | 89 | | 0 |

| | | | | |
|---|---|---|---|---|
| *)= Vergleichsbeispiel | | | | |

**Tabelle 3**

| | | | | | | | | |
|---|---|---|---|---|---|---|---|---|
| Durchgeführte Versuche mit 3-Methylthiopropionaldehyd (MMP) unter verschiedenen Bedingungen | | | | | | | | |
| 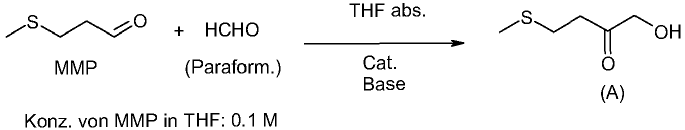 | | | | | | | | |

| **Beisp.** | **MMP (mmol)** | **H₂CO/ MMP (mol)** | **Katalysator (mol %)** | **Base (Base/ Cat, mol)** | **T [°C]** | **t [h]** | **Umsatz von MMP, %** | **Ausbeute (A) (GC, %)** |
|---|---|---|---|---|---|---|---|---|
| 14*) | 5 | 1 | H₂IMes.HBr (2) | Et₃N (10) | 60 | 16 18 | 50 52 | 48 46 |
| 15*) | 1 | 2 | 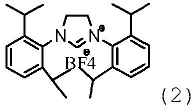 | DBU (8) | 60 | 4 | 86 | 44 |
| 16*) | 1 | 2 | H₂IMes.HBF₄ (2) | DBU (8) | 60 | 4 | 93 | 50 |
| 17*) | 1 | 2 | H₂IMes.HBr (2) | DBU (8) | 60 | 4 | 93 | 57 |
| 18*) | 1 | 2 | H₂IMes.HBF₄ (2) | DBU (8) | 50 | 4 | 93 | 70 |
| 19*) | 1 | 2 | H₂IMes.HBF4 (2) | NaOAc (8) | 50 | 5 | 95 | 75 |
| 20*) | 1 | 1 | H₂IMes.HBF₄ (1) | DBU (8) | RT | 27 | 87 | 62 |
| 21*) | 1 | 1 | H₂IMes.HBF₄ (2) | DBU (8) | RT | 27 | 97 | 68 |
| 22*) | 2 | 2 | H₂IMes.HBF₄ (2) | DBU (8) | RT | 20 | 94 | 75 |
| 23*) | 20 | 2 | H₂IMes.HBF₄ (2) | DBU (8) | RT | 17 20 | 91 94 | 72 75 |
| 24*) | 1 | 2 | H₂IMes.HBF₄ (0.5) | DBU (8) | RT | 20 | 93 | 77 |
| 25*) | 1 | 2 | H₂IMes.HBF₄ (1) | DBU (8) | RT | 27 | 93 | 80 |
| 26*) | 1 | 2 | H₂IMes.HBF₄ (1) | DBU (8) | RT | 8 | 92 | 81 |
| 27 | 1 | 2 | H₂IMes.CO₂ (2) | | RT | 20 | 96 | 81 |
| 28 | 1 | 2 | H₂IMes.CO₂ (1) | ohne Base | RT | 8 22 | 96 97 | 92 93 |
| 29 | 1 | 2 | H₂IMes.CO₂ (0.7) | | RT | 22 | 96 | 91 |
| 30 | 1 | 2 | H₂IMes.CO₂ (0.7) | | RT | 22 | 90 | 90 |
| 31 | 1 | 2 | H₂IMes.CO₂ (0.3) | | RT | 22 | 85 | 81 |
| 32 | 1 | 2 | IMes.CO₂ (1) | | RT | 20 | 57 | 0 |

| | | | | | | | | |
|---|---|---|---|---|---|---|---|---|
| *)= Vergleichsbeispiel | | | | | | | | |

**Tabelle 4**

| | | | | | | |
|---|---|---|---|---|---|---|
| Durchgeführte Versuche mit verschiedenen Aldehyden | | | | | | |
| 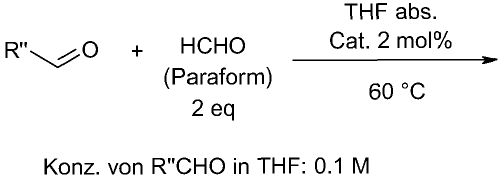 | | | | | | |

| **Beisp.** | **Aldehyd** | **Katalysator** | **t [h]** | **Umsatz von RCHO, %** | **Hauptprodukt** | **Ausbeute (GC, %)** |
|---|---|---|---|---|---|---|
| 33*) | Pentanal | H₂IMes.HBF₄ + DBU (1:8 mol) | 20 | 69 | 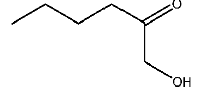 | 52 |
| | | | 44 | 72 | | 50 |
| 34 | | H₂IMes.CO₂ | 20 | 87 | | 67 |
| | | | 44 | 96 | | 70 |
| 35*) | Benzaldehyd | H₂IMes.HBF₄ + DBU (1:8 mol) | 20 | 42 | 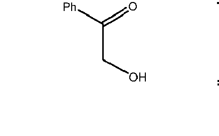 | 33 |
| | | | 44 | 40 | | 30 |
| 36 | | H₂IMes.CO₂ | 20 44 | 44 47 | | 35 35 |
| 37*) | Phenylpropionaldehyd | H₂IMes.HBF₄ + DBU (1:8 mol) | 20 | 90.5 | 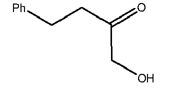 | 81 |
| | | | 44 | 92 | | 80 |
| 38 | | H₂IMes.CO₂ | 20 | 92 | | 80 |
| | | | 44 | 94 | | 81 |
| 39*) | Octanal | H₂IMes.HBF₄ + DBU (1:8 mol) | 20 | 65 | 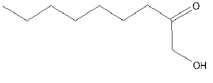 | 53 |
| | | | 44 | 68 | | 55 |
| 40 | | H₂IMes.CO₂ | 20 | 56 | | 48 |
| | | | 44 | 65 | | 55 |
| 41*) | Cyclohexanaldehyde | H₂IMes.HBF₄ + DBU (1:8 mol) | 20 | 32 | 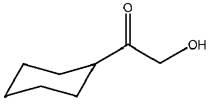 | 26 |
| | | | 88 | 36 | | 30 |
| 42 | | H₂IMes.CO₂ | 20 | 33 | | 27 |
| | | | 44 | 40 | | 28 |
| 43*) | 5-Methylfurfural (RT) | H₂IMes.HBF₄ + DBU (1:8 mol) | 20 | 67 | 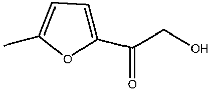 | 58 |
| | | | 44 | 71 | | 60 |
| 44 | | H₂IMes.CO₂ | 20 | 68 | | 58 |
| | | | 44 | 70 | | 62 |

| | | | | | | |
|---|---|---|---|---|---|---|
| *)= Vergleichsbeispiel | | | | | | |

Erklärung zu Tabellen 3, 4: 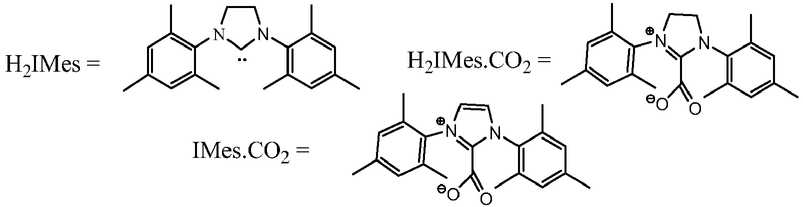 NMR- und MS-Daten von ausgewählten Produkten:

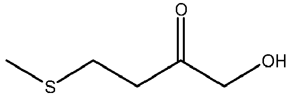

### 1-Hydroxy-4-(methylthio)butan-2-on

¹H-NMR (300 MHz, CDCl₃, 25 °C) : δ = 2.06 (s, 3 H, CH₃), 2.06-2.82 (m, 4 H, CH₂CH₂), 3.03 (bs, 1 H, OH), 4.22 (s, 2 H, CH₂OH); ¹³C-NMR (100 MHz, CDCl₃, 25 °C) : δ = 15.8 (CH₃), 27.7 (SCH₂), 38.2 (CH₂), 68.5 (CH₂OH), 208.1 (C=O); MS (70 eV): m/z (%) : 134 (13) [M⁺], 119 (2) [M⁺ - CH₃], 106 (12), 103 (13) [M⁺ - CH₂OH], 75 (40) [CH₃SCH₂CH₂⁺], 61 (100) [CH₃SCH₂⁺]; Elem. anal.: calcd. for C₅H₁₀O₂S: C, 44.75; H, 7.51; S, 23.89, found: C, 44.77; H, 7.46; S, 24.07; mp 36 °C.

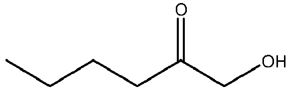

### 1-Hydroxyhexan-2-on

¹H-NMR (300 MHz, CDCl₃, 25 °C): δ = 0.85 (t, ³*J* (H,H) = 7.4 Hz, 3 H; CH₃), 1.29 (m, ³*J* (H, H) = 7.4 Hz, 2 H; 5-C*H*₂), 1.55 (m, ³*J* (H, H) = 7.4 Hz, 2 H, 4-C*H₂*)*,* 2.35 (t, ³*J* (H, H) = 7.4 Hz, 2 H, 3-C*H₂*), 3.13 (bs, 1 H, *OH*), 4.18 (s, 2 H, C*H₂*OH); ¹³C-NMR (100 MHz, CDCl₃, 25 °C) : δ = 13.7 (CH₃), 22.3 (5-CH₂), 25.8 (4-CH₂), 38.1 (3-CH₂), 68.1 (CH₂OH), 209.9 (C=O) ; MS (70 eV) : m/z (%) : 116 (4) [M⁺], 86 (4), 85 (67) [M⁺-CH₂OH], 57 (100) [M⁺ - COCH₂OH], 55 (11), 41 (75), 39 (22).

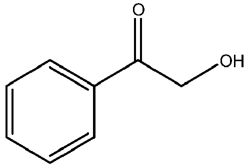

### 2-Hydroxy-1-phenylethanon (2-Hydroxy-acetophenon)

¹H-NMR (300 MHz, CDCl₃ 25 °C) : δ = 4.79 (s, 2 H; C*H*₂), 5.6 (bs, 1 H; O*H*), 7.55-7.97 (m, 5 H; Ph) ppm; ¹³C-NMR (100.6 MHz, CDCl₃, 25 °C) : δ = 67.7 (CH₂), 128.7, 128.8 (x 2), 133.2 (x 2), 136.7, 198.4 (C=O) ppm;. MS (70 eV), *m*/*z* (%) : 136 (6) [M⁺], 106 (8), 105 (100), [M⁺ - CH₂OH], 77 (60) [Ph⁺], 51 (16).

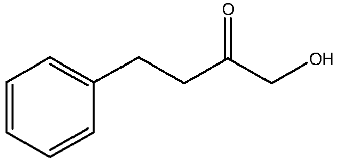

### 1-Hydroxy-3-phenylpropan-2-on

¹H-NMR (300 MHz, CDCl₃ 25 °C): δ = 2.60 (t, ³*J* (H,H) = 7.7 Hz, 2 H; 4-C*H*₂), 2.84 (t, ³*J* (H, H) = 7.7 Hz, 2 H; 3-C*H*₂), 3.19 (s, 1 H; O*H*), 4.06 (s, 2 H; 1-C*H*₂), 7.05-7.12 (m, 5 H; Ph) ppm; ¹³C-NMR (100.6 MHz, CDCl₃, 25 °C) : δ = 29.5 (4-C), 39.9 (3-C), 68.4 (1-C), 126.4, 128.3 (x 2), 128.7 (x 2), 140.3, 209.1 (C=O) ppm;. MS (70 eV): m/z (%) : 164 (4) [M⁺], 146 (4), 133 (35) [M⁺ - CH₂OH], 105 (73) [M⁺ - COCH₂OH], 79 (12), 78 (10), 77 (22) [Ph⁺], 65 (13), 51 (12), 39 (10).

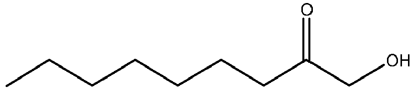

### 1-Hydroxynonan-2-on

¹H-NMR (300 MHz, CDCl₃, 25 °C) : δ = 0.82 (m, ³*J* (H,H) = 6.7 Hz, 3 H; C*H*₃), 1.20 (m, 8 H; 5- - 8-CH₂), 1.56 (m, ³*J* (H, H) = 7.4 Hz, 2 H, 4-C*H*₂)*,* 2.33 (t, ³*J* (H, H) = 7.4 Hz, 2 H, 3-C*H*₂), 3.18 (bs, 1 H, *OH*), 4.17 (s, 2 H, C*H*₂OH); ¹³C-NMR (100 MHz, CDCl₃, 25 °C) : δ = 14.0 (CH₃), 22.6 (8-CH₂), 23.7 (7-CH₂), 28.9 (6-CH₂), 29.1 (5-CH₂), 31.6 (4-CH₂), 38.4 (3-CH₂), 68.1 (CH₂OH), 210.0 (C=O) ; MS (70 eV): m/z (%): 158 (3) [M⁺], 127 (22) [M⁺ - CH₂OH], 111 (18), 95 (2), 83 (8), 69 (100), 57 (68), 55 (47), 43 (48), 41 (43).

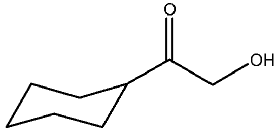

### 1-Cyclohexyl-2-hydroxyethanon

¹H-NMR (300 MHz, CDCl₃, 25 °C) : δ = 1.1-1.4 (m, 6 H; C*H*₂), 1.6-1.8 (m, 4 H; C*H*₂), 2.32 (m, ³*J* (H,H) = 11.4, 3.3 Hz, 1 H, C*H*), 3.22 (bs, 1 H, *OH*), 4.23 (s, 2 H, C*H*₂OH) ppm; ¹³C-NMR (100.6 MHz, CDCl₃, 25 °C) : δ = 25.4 (x 2), 25.6 (x 2), 47.0 (CH), 66.4 (CH₂OH), 212.7 (C=O) ppm;. MS (70 eV), *m*/*z* (%) : 142 (2) [M⁺], 111 (27) [M⁺ - CH₂OH], 84 (7), 83 (100) [M⁺ - COCH₂OH], 67 (12), 55 (91), 41 (48), 39 (28).

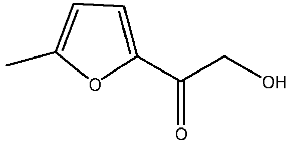

### 2-Hydroxy-1-(5-methylfuran-2-yl)ethanon

¹H-NMR (300 MHz, CDCl₃, 25 °C) : δ = 2.34 (d, ⁴*J* (H,H) = 1.0, 3 H; C*H*₃), 3.28 (bs, 1 H; O*H*), 4.67 (s, 2 H; C*H*₂), 6.14 (dq, J (H,H) = 3.6, 1.0, 1 H; 4'-H), 7.14 (d, ³*J* (H,H) = 3.6, 1 H; 3'-H) ppm; ¹³C-NMR (100.6 MHz, CDCl₃, 25 °C): δ = 14.0 (CH₃), 64.6 (CH₂), 109.3 (4'-C), 119.8 (3'-C), 148.7 (5'-C), 158.6 (2'-C), 186.7 (C=O) ppm;. MS (70 eV), *m*/*z* (%) : 140 (20) [M⁺], 109 (100) [M⁺ - HOCH₂], 95 (2), 81 (1), 65 (1), 53 (33), 43 (7).

NMR Daten vom neuen Katalysator (H₂IMes.CO₂)

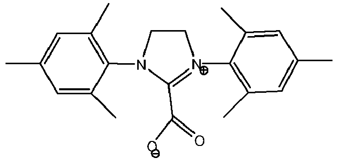

### 1,3-Dimesityl-4,5-dihydro-1H-imidazol-3-ium-2-carboxylat

¹H-NMR (300 MHz, DMSO-d₆, 25 °C) : δ = 2.25 (s, 6 H; C*H*₃), 2.36 (s, 12 H; C*H*₃), 4.43 (s, 4 H; C*H*₂), 6.98 (s, 4 H; Arom.) ppm; ¹³C-NMR (300.6 MHz, DMSO-d₆, 25 °C) : δ = 20.5 (4 CH₃), 24.1 (2 CH₃), 52.8 (2 CH₂), 132.5 (C-3', C-5'), 134.7 (C-4'), 139.8 (C-2', C-6'), 142.5 (C-1'), 155.5 (C-2), 167.7 (CO₂) ppm.

## Patentansprüche

1. Verwendung von Verbindungen der allgemeinen Formel III: 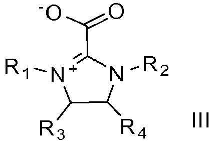 als Katalysator zur Herstellung von α-Hydroxyketonen mittels Acyloinaddition von Aldehyden, wobei R¹ und R² gleich oder verschieden sind und C₆-C₁₀- Aryl, Heteroaryl, verzweigtes oder unverzweigtes ggf. ein- oder mehrfach mit C₁-C₄-Alkyl oder mit Heteroalkyl subsitutiertes C₁- C₁₀-Alkyl, C₃-C₆-Cycloalkyl oder ein- oder mehrfach mit C₁-C₄-Alkyl substituiertes C₆-C₁₀-Aryl bedeuten und R³ und R⁴ gleich oder verschieden sind und Wasserstoff, ggf.verzweigtes C₁-C₆-Alkyl-, C₃-C₆-Cycloalkyl-, C₆-C₁₀-Aryl- oder Heteroaryl bedeuten.

2. Verbindung der Formel IV 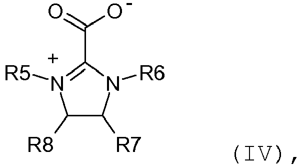 wobei R⁵ und R⁶ gleich oder verschieden sind und C₆-C₁₀-Aryl, ggf. ein- oder mehrfach mit C₁-C₄-Alkyl substituiertes C₁-C₁₀-Alkyl oder C₃-C₆-Cycloalkyl bedeuten und R⁷ und R⁸ gleich oder verschieden sind und Wasserstoff oder ggf. verzweigtes C₁-C₆-Alkyl bedeuten, mit der Maßgabe, dass wenn R⁷ und R⁸ gleich Wasserstoff sind, R⁵ und R⁶ nicht gleichzeitig Phenyl sind.

3. Verwendung der Verbindung IV 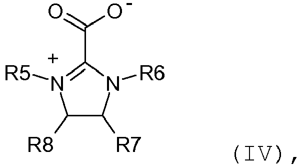 wobei R⁵ und R⁶ gleich oder verschieden sind und C₆-C₁₀-Aryl, ein- oder mehrfach mit C₁-C₄-Alkyl substituiertes C₆-C₁₀-Aryl, ggf. ein- oder mehrfach mit C₁-C₄-Alkyl substituiertes C₁-C₁₀-Alkyl oder C₃-C₆-Cycloalkyl bedeuten und R⁷ und R⁸ gleich oder verschieden sind und Wasserstoff oder ggf. verzweigtes C₁-C₆-Alkyl bedeuten, mit der Maßgabe, dass wenn R⁷ und R⁸ gleich Wasserstoff sind, R⁵ und R⁶ nicht gleichzeitig Phenyl sind, als Katalysator zu Herstellung von α-Hydroxyketonen mittels Acyloinaddition von Aldehyden.

4. Verwendung der Verbindung IV gemäß Anspruch 3, **dadurch gekennzeichnet, dass** der ein- oder mehrfach C₁-C₄-Alkyl substituierte C₆-C₁₀-Aryl-Rest Mesityl ist.

5. Verfahren zur Herstellung von α-Hydroxyketonen der allgemeinen Formel I: 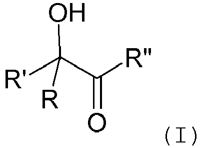 durch Umsetzung einer Carbonylverbindung der allgemeinen Formel RR'C=O mit einer Carbonylverbindung der allgemeinen Formel HR''C=O, in Gegenwart eines Katalysators der Formel III oder IV gemäß Anspruch 1-4,
wobei R und R' gleich oder verschieden sind und H oder einen geradkettigen oder verzweigten und ggf. substituierten C₁-C₁₂-Alkyl-Rest bedeuten und R'' = H₃CSCH₂CH₂, t-Butyl, n-Butyl, sek-Butyl, n-Propyl, i-Propyl, ein gegebenfalls hetereoatomsubstituiertes C₆-C₁₈-Aryl, Heteroaryl, C₆-C₁₈-Arylalkyl oder Heteroalkyl ist.

6. Verfahren gemäß Anspruch 5, **dadurch gekennzeichnet, dass**
R = R'=H und
R'' = CH₃SCH₂CH₂ ist oder,
dass
R = H,
R' = CH₃SCH₂CH₂ und
R'' = H ist oder,
dass
R = H
R' = CH₃ und
R'' = CH₃SCH₂CH₂ ist oder,
dass
R = H
R' = C₂H₅ und
R'' = CH₃SCH₂CH₂ ist.

7. Verfahren gemäß einem der Ansprüche 5 oder 6, **dadurch gekennzeichnet, dass** der Katalysator in einer Konzentration von 0,1-5 Mol% bezogen auf den ggf. im Unterschuß eingesetzten Aldehyd verwendet wird.

8. Verfahren gemäß einem der Ansprüche 5 bis 7, **dadurch gekennzeichnet, dass** die Reaktion bei Temperaturen von -20 °C bis 100°Cdurchgeführt wird.

9. Verfahren gemäß Anspruch 8, **dadurch gekennzeichnet**, das die Reaktion bei Temperaturen von 15°C bis 80°C durchgeführt wird.

10. Verfahren gemäß einem der Ansprüche 5 bis 9 **dadurch gekennzeichnet, dass** die Reaktion unter einem Partialdruck von 0,1-20 bar Kohlendioxid durchgeführt wird.

11. Verfahren zur Herstellung von Katalysatoren gemäß der Ansprüche 1 oder 2
**dadurch gekennzeichnet, dass** man eine entsprechende Verbindung der allgemeinen Formel X oder XI 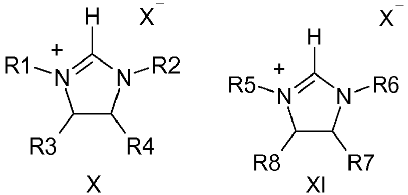 in Gegenwart einer Base mit CO₂ umsetzt, wobei X = Cl, Br, I, pCH₃C₆H₄SO₃, BF₄, PF₆, CH₃SO₃ ist.

12. Verfahren gemäß Anspruch 11, **dadurch gekennzeichnet, dass** Basen verwendet werden, deren korrespondierende Säure einen pKa > 8 aufweisen.

13. Verfahren gemäß Anspruch 12, **dadurch gekennzeichnet, dass** als Base Alkali- und Erdalkalicarboxylate, Alkali- und Erdalkalialkoholate, Alkali- und Erdalkalicarbonate oder primäre, sekundäre, tertiäre Amine oder bicyclische Amine eingesetzt werden.

14. Verfahren gemäß Anspruch 13, **dadurch gekennzeichnet, dass** Natriumacetat, Kalium-tert.-butylat oder 1,8-Diazobicyclo[5.4.0]undec-7-en eingesetzt wird.

15. Verfahren gemäß Anspruch 11 bis 14, **dadurch gekennzeichnet, dass** die Reaktion bei Temperaturen von -20 °C bis 100°C durchgeführt wird.

## Claims

1. Use of compounds of the general formula III: 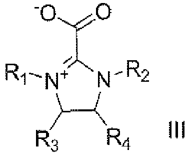 as a catalyst for the preparation of α-hydroxy ketones by means of acyloin addition of aldehydes, where R¹ and R² are the same or different and are each C₆-C₁₀-aryl, heteroaryl, branched or unbranched, optionally mono- or poly-C₁-C₄-alkyl-or -heteroalkyl-substituted C₁-C₁₀-alkyl, C₃-C₆-cycloalkyl or mono- or poly-C₁-C₄-alkyl-substituted C₆-C₁₀-aryl, and R³ and R⁴ are the same or different and are each hydrogen, branched or unbranched C₁-C₆-alkyl, C₃-C₆-cycloalkyl, C₆-C₁₀-aryl or heteroaryl.

2. Compound of the formula IV 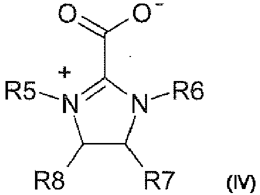 where R⁵ and R⁶ are the same or different and are each C₆-C₁₀-aryl, optionally mono- or poly-C₁-C₄-alkyl-substituted C₁-C₁₀-alkyl or C₃-C₆-cycloalkyl, and R⁷ and R⁸ are the same or different and are each hydrogen or branched or unbranched C₁-C₆-alkyl, with the proviso that, when R⁷ and R⁸ are each hydrogen, R⁵ and R⁶ are not both phenyl.

3. Use of the compound IV, 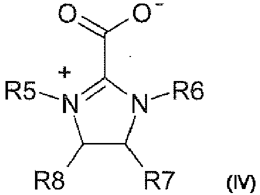 where R⁵ and R⁶ are the same or different and are each C₆-C₁₀-aryl, mono- or poly-C₁-C₄-alkyl-substituted C₆-C₁₀-aryl, optionally mono- or poly-C₁-C₄-alkyl-substituted C₁-C₁₀-alkyl or C₃-C₆-cycloalkyl, and R⁷ and R⁸ are the same or different and are each hydrogen or branched or unbranched C₁-C₆-alkyl, with the proviso that, when R⁷ and R⁸ are each hydrogen, R⁵ and R⁶ are not both phenyl, as a catalyst for preparation of α-hydroxy ketones by means of acyloin addition of aldehydes.

4. Use of the compound IV according to Claim 3, **characterized in that** the mono- or poly-C₁-C₄-alkyl-substituted C₆-C₁₀-aryl radical is mesityl.

5. Process for preparing α-hydroxy ketones of the general formula I: 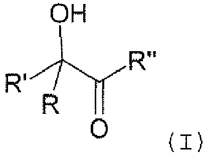 by reacting a carbonyl compound of the general formula RR'C = O with a carbonyl compound of the general formula HR" C = O, in the presence of a catalyst of the formula III or IV according to Claim 1-4,
where R and R' are the same or different and are each H or a straight-chain or branched and optionally substituted C₁-C₁₂-alkyl radical, and R" = H₃CSCH₂CH₂, t-butyl, n-butyl, sec-butyl, n-propyl, i-propyl, an optionally heteroatom-substituted C₆-C₁₀-aryl, heteroaryl, C₆-C₁₈-arylalkyl or heteroalkyl.

6. Process according to Claim 5, **characterized in that**
R = R' = H and
R" = CH₃SCH₂CH₂ or
**in that**
R = H,
R' = CH₃SCH₂CH₂ and
R" = H or
**in that**
R = H,
R' = CH₃ and
R" = CH₃SCH₂CH₂ or
**in that**
R = H,
R' = C₂H₅ and
R" = CH₃SCH₂CH₂.

7. Process according to either of Claims 5 and 6, **characterized in that** the catalyst is used in a concentration of 0.1-5 mol% based on the aldehyde which may be used in deficiency.

8. Process according to one of Claims 5 to 7, **characterized in that** the reaction is performed at temperatures of -20°C to 100°C.

9. Process according to Claim 8, **characterized in that** the reaction is performed at temperatures of 15°C to 80°C.

10. Process according to one of Claims 5 to 9, **characterized in that** the reaction is performed under a partial pressure of 0.1 - 20 bar of carbon dioxide.

11. Process for preparing catalysts according to Claims 1 or 2,
**characterized in that**
a corresponding compound of the general formula X or XI 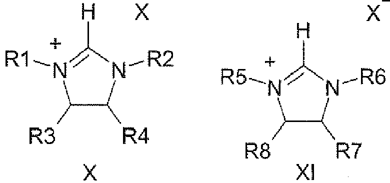 is reacted with CO₂ in the presence of a base, where X = Cl, Br, I, pCH₃C₆H₄SO₃, BF₄, PF₆, CH₃SO₃.

12. Process according to Claim 11, **characterized in that** bases whose corresponding acid has a pKa > 8 are used.

13. Process according to Claim 12, **characterized in that** the bases used are alkali metal and alkaline earth metal carboxylates, alkali metal and alkaline earth metal alkoxides, alkali metal and alkaline earth metal carbonates or primary, secondary, tertiary amines or bicyclic amines.

14. Process according to Claim 13, **characterized in that** sodium acetate, potassium tert-butoxide or 1,8-diazabicyclo[5.4.0]undec-7-ene is used.

15. Process according to Claim 11 to 14, **characterized in that** the reaction is performed at temperatures of -20°C to 100°C.

## Revendications

1. Utilisation de composés de formule générale III : 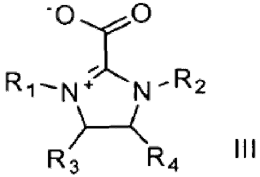 en tant que catalyseur pour la fabrication d'α-hydroxycétones par addition acyloïne d'aldéhydes, R¹ et R² étant identiques ou différents, et signifiant aryle en C₆-C₁₀, hétéroaryle, alkyle en C₁-C₁₀ ramifié ou non ramifié, éventuellement substitué une ou plusieurs fois avec alkyle en C₁-C₄ ou avec hétéroalkyle, cycloalkyle en C₃-C₆, ou aryle en C₆-C₁₀ substitué une ou plusieurs fois avec alkyle en C₁-C₄, et R³ et R⁴ étant identiques ou différents, et signifiant hydrogène, alkyle en C₁-C₆ éventuellement ramifié, cycloalkyle en C₃-C₆, aryle en C₆-C₁₀ ou hétéroaryle.

2. Composé de formule IV 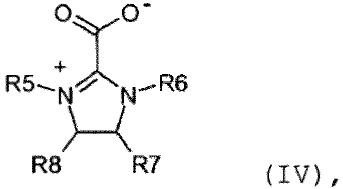 dans laquelle R⁵ et R⁶ sont identiques ou différents, et signifient aryle en C₆-C₁₀, alkyle en C₁-C₁₀ éventuellement substitué une ou plusieurs fois avec alkyle en C₁-C₄, ou cycloalkyle en C₃-C₆, et R⁷ et R⁸ sont identiques ou différents, et signifient hydrogène ou alkyle en C₁-C₆ éventuellement ramifié, à condition que lorsque R⁷ et R⁸ signifient hydrogène, R⁵ et R⁶ ne signifient pas simultanément phényle.

3. Utilisation du composé IV 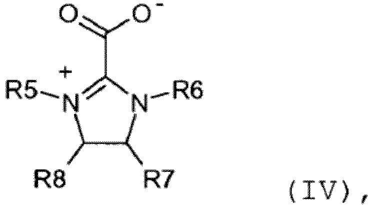 dans laquelle R⁵ et R⁶ sont identiques ou différents, et signifient aryle en C₆-C₁₀, aryle en C₆-C₁₀ substitué une ou plusieurs fois avec alkyle en C₁-C₄, alkyle en C₁-C₁₀ éventuellement substitué une ou plusieurs fois avec alkyle en C₁-C₄, ou cycloalkyle en C₃-C₆, et R⁷ et R⁸ sont identiques ou différents, et signifient hydrogène ou alkyle en C₁-C₆ éventuellement ramifié, à condition que lorsque R⁷ et R⁸ signifient hydrogène, R⁵ et R⁶ ne signifient pas simultanément phényle, en tant que catalyseur pour la fabrication d'α-hydroxycétones par addition acyloïne d'aldéhydes.

4. Utilisation du composé IV selon la revendication 3, **caractérisée en ce que** le radical aryle en C₆-C₁₀ substitué une ou plusieurs fois par alkyle en C₁-C₄ est mésityle.

5. Procédé de fabrication d'α-hydroxycétones de formule générale I : 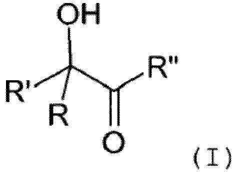 par mise en réaction d'un composé carbonyle de formule générale RR'C=O avec un composé carbonyle de formule générale HR''C=O, en présence d'un catalyseur de formule III ou IV selon les revendications 1 à 4,
R et R' étant identiques ou différents, et signifiant H ou un radical alkyle en C₁-C₁₂ linéaire ou ramifié et éventuellement substitué, et R'' = H₃CSCH₂CH₂, t-butyle, n-butyle, sec-butyle, n-propyle, i-propyle, aryle en C₆-C₁₈ éventuellement substitué par un hétéroatome, hétéroaryle, arylalkyle en C₆-C₁₈ ou hétéroalkyle.

6. Procédé selon la revendication 5, **caractérisé en ce que**
R = R' = H, et
R'' = CH₃SCH₂CH₂, ou
**en ce que**
R = H,
R' = CH₃SCH₂CH₂ et
R'' = H, ou
**en ce que**
R = H,
R' = CH₃ et
R'' = CH₃SCH₂CH₂, ou
**en ce que**
R = H,
R' = C₂H₅ et
R''" = CH₃SCH₂CH₂.

7. Procédé selon l'une quelconque des revendications 5 ou 6, **caractérisé en ce que** le catalyseur est utilisé en une concentration de 0,1 à 5 % en moles, par rapport à l'aldéhyde éventuellement utilisé en déficit.

8. Procédé selon l'une quelconque des revendications 5 à 7, **caractérisé en ce que** la réaction est réalisée à des températures de -20 °C à 100 °C.

9. Procédé selon la revendication 8, **caractérisé en ce que** la réaction est réalisée à des températures de 15 °C à 80 °C.

10. Procédé selon l'une quelconque des revendications 5 à 9, **caractérisé en ce que** la réaction est réalisée à une pression partielle de 0,1 à 20 bar de dioxyde de carbone.

11. Procédé de fabrication de catalyseurs selon les revendications 1 ou 2, **caractérisé en ce qu'**un composé correspondant de formule générale X ou XI 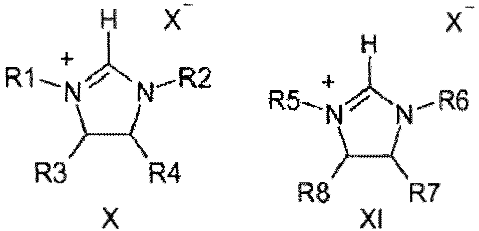 est mis en réaction en présence d'une base avec CO₂, avec X = Cl, Br, I, pCH₃C₆H₄SO₃, BF₄, PF₆, CH₃SO₃.

12. Procédé selon la revendication 11, **caractérisé en ce que** des bases dont les acides correspondants présentent un pKa > 8 sont utilisées.

13. Procédé selon la revendication 12, **caractérisé en ce que** des carboxylates alcalins et alcalino-terreux, des alcoolates alcalins et alcalino-terreux, des carbonates alcalins et alcalino-terreux, ou des amines primaires, secondaires, tertiaires ou des amines bicycliques sont utilisés en tant que base.

14. Procédé selon la revendication 13, **caractérisé en ce que** de l'acétate de sodium, du tert.-butylate de potassium ou du 1,8-diazobicyclo[5.4.0]undéc-7-ène est utilisé.

15. Procédé selon les revendications 11 à 14, **caractérisé en ce que** la réaction est réalisée à des températures de -20 °C à 100 °C.
